Europäisches Patentamt

European Patent Office

Office européen des brevets

(19)

(11) **EP 0 750 189 A1**

(12) **EUROPÄISCHE PATENTANMELDUNG**

(43) Veröffentlichungstag:
27.12.1996 Patentblatt 1996/52

(51) Int. Cl.⁶: **G01N 27/00**, G01N 33/18

(21) Anmeldenummer: 96108981.0

(22) Anmeldetag: 05.06.1996

(84) Benannte Vertragsstaaten:
**AT CH DE FR GB IT LI NL**

(30) Priorität: **20.06.1995 DE 19522278**

(71) Anmelder: **Buna Sow Leuna Olefinverbund GmbH**
**D-06258 Schkopau (DE)**

(72) Erfinder:
• **Adler, Bernhard, Prof. Dr.**
**06132 Halle (DE)**
• **Auge, Jörg**
**39326 Wolmirstedt (DE)**
• **Hartmann, Jens, Dr.**
**06144 Halle (DE)**
• **Hauptmann, Peter, Prof. Dr.**
**39326 Hermsdorf (DE)**
• **Rösler, Steffen**
**39108 Magdeburg (DE)**

(54) **Verfahren zum Nachweis amphiphiler Stoffe in wässriger Matrix und Vorrichtung zur Durchführung des Verfahrens**

(57)     Die Erfindung betrifft die Anwendung eines QMB-Flüssigkeitssensors zur Routine-Tensidanalytik sowohl in der off line-Laboranalytik als auch als on line-Meßgerät für in situ-Messungen in Abwässerkanälen bzw. biologischen Kläranlagen. Erfindungsgemäß wird die Aufgabe durch entsprechend konstruierte Durchflußzellen gelöst. Das Durchströmen dieser Zellen mit Realproben erfolgt so, daß Tenside aus der Abwassermatrix selbständig an die Metallkontaktion des Schwingquarzes diffundieren, die dort befindliche Wasserschicht verdrängen und die damit verbundene Masseänderung in Form einer Frequenzänderung als Analysensignal genutzt wird.

Fig.3

EP 0 750 189 A1

**Beschreibung**

Die Erfindung findet Anwendung zum selektiven Nachweis amphiphiler Stoffe in einer wäßrigen Matrix, insbesondere einer heterogen zusammengesetzten Abwassermatrix in vito oder in situ.

Der Nachweis von Tensiden im Abwasser stellt ein diffiziles analytisches Problem dar. Es existieren bisher keine on line-Systeme, die solche Überwachungen in den erlaubten Konzentrationsbereichen gewährleisten und zudem wirtschaftlich in der Praxis angewendet werden können.

Bekannt ist, daß amphiphile Stoffe wie z.B. technische Tenside in der Routineanalytik aus wäßriger Phase durch Extraktionen mit organischen Lösungsmitteln angereichert werden und über Adduktbildung als farbige Komplexe mit Methylenblau oder Cobaltisothiocyanat photometrisch dedizierbar sind (DIN 38409, DIN EN 903). Diese Anreicherungen sind nicht nur zeit-und kostenintensiv, sondern stellen ein zum Teil hohes gesundheitliches Risiko dar, wenn z.B. mit Chloroform extralliert werden muß.

Zum Nachweis von Analytmolekülen in Flüssigkeiten sind chemische Resonanzsensoren, basierend auf dem Prinzip der Quarzkristall-Mikrobalance (QMB), bekannt. Der Sensor besteht aus einem dünnen Quarzsubstrat mit beidseitig aufgebrachten Goldelektroden. Durch das Erzeugen eines elektrischen Feldes wird der Quarz aufgrund seiner piezoelektrischen Eigenschaften zu Dickenscherschwingungen angeregt. Eine zusätzliche Massebelegung führt zu einer Verschiebung der Resonanzfrequenz des Quarzes. Als fundamentaler Zusammenhang zwischen der Frequenzänderung $\Delta f$ und der Masseänderung $\Delta m$ [G. Sauerbrey, Z. Phys. 155 (1959) 206] gilt:

$$-\Delta f \sim f_o{}^2 \cdot \Delta m \qquad\qquad (1)$$

mit $\Delta f$ Frequenzänderung, $f_o$ Resonanzfrequenz und $\Delta m$ Masseänderung.

Soll der Quarz als massensensitives Sensorelement zum Nachweis von speziellen Analyten verwendet werden, erfolgt gewöhnlich eine Beschichtung der Elektroden mit hierfür geeigneten sensitiven chemischen Schichtmaterialien. Die Wechselwirkungen der Analytmoleküle mit den Sensorschichten können unterschiedlicher Art sein und ermöglichen die Realisierung sowohl reversibler als auch irreversibler Signale, Nachweisgrenzen von $10^{-9}$ g sind dabei erreichbar.

Beim Einsatz der Quarze in Flüssigkeiten wird der zuvor genannte Zusammenhang von Masse-und Frequenzänderung überlagert durch additive Komponenten, die vorrangig durch die stofflichen Kenngrößen der Flüssigkeit determiniert sind [K.K. Kanazawa, J.G. Gordon; Anal.Chem. 57 (1959) 1771]. Beeinflußt wird das Schwingungsverhalten des Quarzes durch den viskosen Flüssigkeitskontakt der Quarzoberfläche [S.J. Martin, V.E. Granstaff, G.C. Frye; Anal. Chemie 63 (1991) 2272], durch Oberflächenrauhigkeiten [S.J. Martin, G.C. Frye, K.O. Wessendorf; Sens.Actuators A 44 (1994) 209] , durch die eigentliche Massebelegung und bei beidseitigem Kontakt des Quarzes mit der Flüssigkeit zusätzlich durch Leitfähigkeit und dielektrisches Verhalten der Flüssigkeit [C. Barnes; Sens. Actuators A 30 (1992) 197].

Bekannt ist, daß Quarzmikrobalance-Sensoren zum analytischen Nachweis von biochemischen Wirksubstanzen in wässrigen Lösungen (Biosensoren) dienen. Als spezifische Beschichtungen werden Enzyme [G.G. Guibault, J.H. Luong; J. of Biotechnolog. 9 (1988) 1], Antigene [C. Kößlinger, S. Drost, F. Aberl, H. Wolf, S. Koch und P. Woias; Biosens. Bioelectronics 7 (1992) 398], DNA-Teilstrukturen [Y. Okahata, Y. Matsunobu, K. Ijiro, M. Mukae, A. Murakami, K. Makino; J. Am. Chem. Soc. 114 (1992) 8299] oder Rezeptoren [H. Ebato, J.N. Herron, W. Müller, Y. Okahata, H.Ringsdorf, P. Suci; Angew. Chem. 104 (1992) 1064] verwendet.

Die Nutzung des QMB-Prinzips als Biosensoren ist wegen der großen Molmasse des Bioanalyts und der spezifischen Wechselwirkung zwischen Biokomponente und Analyt von besonderem Vorteil. Aber auch die Detektion von chemischen Substanzen mittels Lipid-beschichteten Sensoren [Y. Okahata, H. Ebato, X, Ye; J. Chem. Soc., Chem. Commun. (1988) 1037] und Chlolesteryl-Schichten durch lipophile Anreicherungen [J. Auge, P. Hauptmann, F. Eichelbaum, S. Rösler; Sens. Actuators B 19 (1994) 518] ist beschrieben. Ferner liegt eine Publikation über Lipid-beschichteter QMB-Sensoren zum Nachweis toxischer und pharmakologischer Aktivitäten vor [Y. Okahata, H. Ebato; Anal. Chem. 63 (1991) 203].

Zudem ist es möglich, auch in organischen Lösungsmitteln chemische Stoffe (z.B. Verunreinigungen) mittels QMB-Sensoren nachzuweisen [J. Auge, J. Hartmann, P. Hauptmann, S. Rösler; Exp. Techn. Phys. 40 (1994) 27].

Eine Sensoranordnung zum Nachweis eines Stoffes in einer Flüssigkeit, speziell der elektronische Teil der Anordnung, ist unter DE 4.028.500 (1992) und EP 473901 (1990) patentiert worden. Speziell die Bestimmung der Konzentration von Schwefelsäure in Akkusäuren wird in einer ähnlich gebauten Anordnung durch das WO 9306452 (1993) geschützt. Eine Apparatur zur Messung von Dichten in Flüssigkeiten nach dem QMB-Prinzip wird durch das Patent SU 688865 (1978) gesichert.

Der Erfindung liegt die Aufgabe zugrunde, amphiphile Stoffe in wäßriger Matrix ohne vorherige An- oder Abreicherungsoperationen analytisch sowohl im off line- als auch im on line-Betrieb zu erfassen. Die Erfindung soll gewährleisten, daß die amphiphilen Stoffe bis in dem Bereich von 1 ppm analysiert werden, aber es soll auch die Überwachung eines definierbaren Grenzwertes der Konzentration der amphiphilen Stoffe in der wäßrigen Matrix möglich sein.

Die Aufgabe wird durch die Erfindung gemäß der Patentansprüche 1 bis 5 gelöst.

Dabei kommt eine Quarzkristall-Microbalance-Sensorzelle zum Einsatz, deren metallbeschichtete Oberfläche des Schwingquarzes unmittelbar mit dem zu analysierenden wäßrigen Medium in Kontakt gebracht wird. Die Fließgeschwindigkeit des strömenden Mediums, z.B. die zu analysierenden Abwasserproben, ist so zu regulieren, daß die hydratisierten Tensidmoleküle an die Sensoroberfläche diffundieren können. Das sensorische Prinzip greift auf die Eigenschaft des amphiphilen Stoffes, oberflächenaktiv zu sein, zurück. Damit kann einmal auf spezielle Oberflächenbeschichtungen der Metallkontaktierung des Schwingquarzes verzichtet werden. Zum anderen werden alle nicht oberflächenaktiven Stoffe, beispielsweise einer Abwassermatrix selbstwirkend diskriminiert. Dies kann man durch das QMB-Prinzip für den Nachweis der Tenside nutzen. Ist das Wasser tensidhaltig, müssen sich Tenside bzw. Aggregationen von Tensiden auf der Quarzoberfläche anlagern und dadurch die Resonanzfrequenz des Quarzes entscheidend verändern. Als Analysensignal wird die durch den Verdrängungsprozeß von Wasser durch Tensidmoleküle verursachte Masseänderung $\Delta m$ in Form einer Frequerizänderung $\Delta f$ in Hz zur Anzeige gebracht. Bei geringen Konzentrationen von Tensiden, verbunden mit unwesentlichen Änderungen der Viskosität und Dichte der Lösung gilt dabei die Basisgleichung (1). Bei sehr hohen Tensidkonzentrationen und der vermehrten Bildung beispielsweise von Micellen setzt sich das Signal aus Masseanlagerung und Veränderung physikochemischer Eigenschaften der Meßlösung zusammen. Im Falle einer on line-Nutzung dieses sensorischen QMB-Prinzips reicht eine einfache Durchlußzelle wie für den Laborbetrieb nicht aus. Vielmehr muß der Sensor von entgaster Flüssigkeit, die möglichst frei von gröberen Partikeln ist, umspült werden. Erfindungsgemäß wird diese Aufgabe durch eine entsprechende Durchflußzelle gelöst, die die zu analysierende wäßrige Phase entgast, von gröberen Schwebstoffen befreit und für eine hinreichend konstante Strömung sorgen kann.

In einer Beruhigungskammer befindet sich die QMB-Sensorzelle, daneben kann auch ein Multisensorsystem aus unterschiedlichen Sensoren (Sensorarray) untergebracht sein, womit gleichzeitig beispielsweise der pH-Wert, die elektrische Leitfähigkeit, die Temperatur und/oder die Trübung des Analyten gemessen oder überwacht werden können.

In der QMB-Sensorzelle sind ein oder mehrere Schwingquarze so angeordnet, daß eine Seite und damit eine Metallkontaktierung des jeweiligen Quarzes mit der wäßrigen Phase in Kontakt steht, die andere (z.B. durch O-Ring oder Kleben) davon isoliert ist und sich entweder in einer gasförmigen oder einer flüssigen Phase befindet. Vorteilhafterweise wird das sensorische Element Schwingquarz zum Nachweis des Meßeffekes als frequenzbestimmendes Glied einer Oszillatorschaltung eingesetzt. Die Anlagerung von Tensiden an der Oberfläche der Quarz-Metallkontaktierung führt zu einer Veränderung der Resonanzfrequenz des Schwingquarzes, die als frequenzanaloges Ausgangssignal der Oszillatorschaltung leicht erfaßt und weiterverarbeitet werden. Da der Quarz durch den Kontakt mit einer Flüssigkeit sehr stark bedämpft wird und einen starken Güteverlust unterliegt, ist für das Treiben eines Quarzes in Flüssigkeiten eine spezielle Elektronik erforderlich, die eine hinreichend hohe Frequenzstabilität gewährleistet, um den Meßeffekt sicher nachweisen zu können. Herkömmliche Quarzoszillatoren vermögen dies in der Regel nicht. Beschrieben wird eine Schaltung unter Verwendung der Integrierten Schaltung OPA 660 (Burr Brown), die sich insbesondere durch den geringen Bauelementeaufwand, den Verzicht auf weitere frequenzbestimmende Bauelemente (wie Induktivitäten etc.) und dadurch auszeichnet, daß die meßmedienkontaktierende Schwingquarzelektrode elektrisch auf Massepotential liegt.

Die Schaltung OPA 660 stellt eine Kombination dar, da sie die Vorteile einer integrierten Schaltung mit den HF-Eigenschaften nicht rückgekoppelter Verstärker verbindet. Der OPA 660 enthält als IC mit dem Diamond-Transistor (DT) und dem Buffer-Verstärker (DB) zwei eigenständige Schaltungsteile zur Verarbeitung von HF-Signalen bis 500 MHz. Der Diamond-Transistor ist eine spannungsgesteuerte und breitbandige Stromquellen, die eine idealisierte Transistorfunktion bereitstellt. Sie besitzt eine hochohmigen Eingang (Basis), einen niederohmigen Ein-/Ausgang (Ermitter) und einen hochohmigen Sromquellenausgang (Kollektor). Im Gegensatz zum bipolaren Transistor erlaubt die integrierte Schaltung vollen Vierquandrantenbetrieb; es liegen alle ihre Anschlüsse ohne Ansteuerung auf 0V. Alle Arbeitspunkte liegen fest und sind temperaturstabil; die Ermitterschaltung arbeitet jedoch nicht invertierend und die Basisschaltung invertierend. Die Steilheit als Änderung des Ausgangsstromes zur Änderung der Eingangsspannung ist über den gesamten Eingangsspannungsbereich um Größenordnungen konstanter und läßt sich zusätzlich mit einem externen Widerstand programmieren.

Der Daimond-Buffer ist ein zweistufiger, komplementärer Emitterfolger und bildet die ideale Ergänzung zum Diamond-Transtistor. Er entkoppelt nicht belastbare hochohmige Schaltungspunkte ohne Verlust der Bandbreite, verhindert Rückwirkungen vom Ausgang auf die Signalverarbeitung und ermöglicht einen niederohmigen Spannungsausgang zum Treiben eines Kabels, eines niederohmigen Eingangswiderstandes oder eines Rückkopplungsnetzwerkes. Der Daimond-Transistor T1 ist der eigentliche Schwingtransistor, mit dessen Emitter der Schwingquarz und parallel dazu der Widerstand R5 verbunden ist. Über den Kollektorwiderstand R6 ist der Kollektor mit Masse verbunden. Die parallel dazu geschalteten antiparallelen Dioden arbeiten als Amplitudenbegrenzer. Über Widerstand R2 gelangt das Signal an den Eingang des Buffers 1. Der Buffer entkoppelt das Signal und leitet es niederohmig über CI an die Basis von T1 zurück, gleichzeitig wird es über C2 und R7 an die Basis des Ausgangstreiber-Transistors T2 geleitet.

Mit dem Emitterwiderstand R11 wird die Verstärkung der Transistorstufe T2 eingestellt und damit die Größe der Signalausgangsamplitude den Erfordernissen angepaßt. Das verstärkte Signal gelangt über den Widerstand R9 an den Eingang des Buffers 2. Er arbeitet als niederohmige Treiberstufe für die Einkopplung des Signals über C3 in das Kabel. Über eine Auswerteeinheit (Frequenzzähler und Datenspeicher) und einer entsprechenden Software werden die Daten angezeigt und können verarbeitet bzw. ausgedruckt werden.

Die Erfindung wird an folgenden Beispielen näher erläutert. Die Figuren stellen dabei dar:

Fig. 1        - Experimenteller Aufbau eines Meßplatzes mit einer QMB-Meßzelle

Fig. 2        - Tensidsignal der QMB-Meßzelle für das Tensid N 9 bei einer Konzentration von 30 ppm in Wasser

Fig. 3        - Durchflußzelle für eine in situ-Messung im Abwasserkanal

Fig. 4        - Oszillatorschaltung für QMB-Sensor

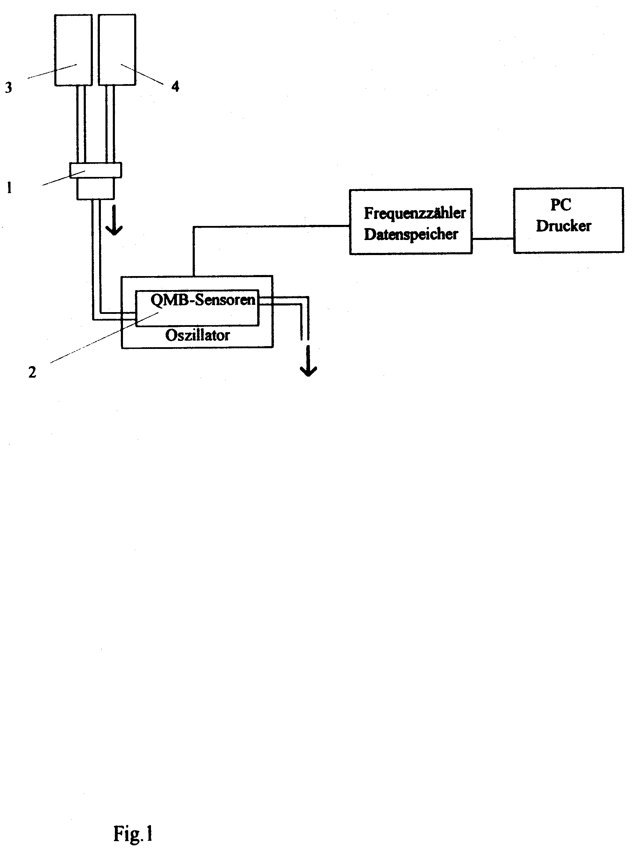

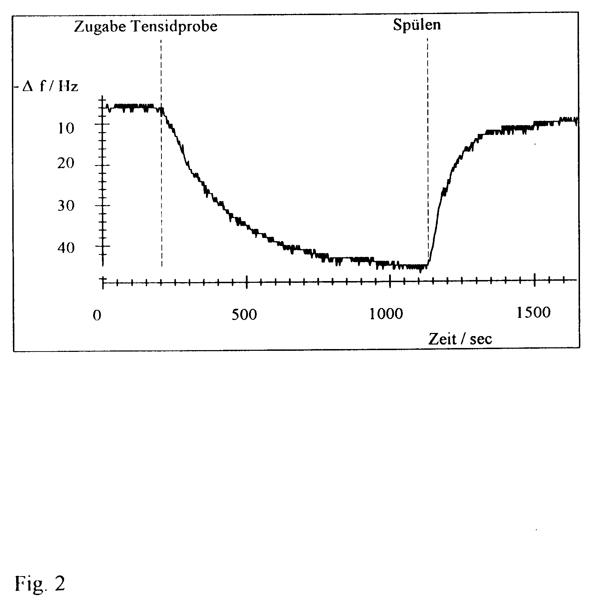

Destilliertes Wasser durchläuft mit konstanter Strömung, durch einen Flowcontroller 1 eingestellt, die QMB-Sensorzelle 2 gemäß Meßanordnung der Fig. 1. Aus einem Vorratsgefäß 3 wird ein Wasser-Trägermedium und aus einem Vorratsgefäß 4 die Analyt-Wasser-Lösung entnommen. Nach Erreichen einer stabilen Grundlinie wird die Zuführung aus Gefäß 1 unterbunden und die wäßrige Analytlösung mit gleicher Strömungsgeschwindigkeit durchgeleitet. Nach ca. $3 - 8 \cdot 10^2$s wird erneut auf Gefäß 3 umgeschaltet, wobei solange gespült werden muß, bis das Grundniveau wieder erreicht ist. Die erreichte Frequenzänderung (Fig. 2) bei Durchlauf des Analyten kann durch eine Kalibrierkurve in Konzentrationswerte umgerechnet werden. Für die Typen NIT 9, NIT 5,5 und NIT F 1315 sowie das AAT sind die Frequenzänderungen für 30 ppm in Tabelle 1 zusammengefaßt.

Tabelle 1

| Frequenzänderungen von Tensiden bei Konzentrationen von jeweils 30 ppm (NIT Nichtionogene Tenside, AAT Anionenaktive Tenside) | | |
|---|---|---|
| Probebezeichng. Tensid | Struktur des Tensids | Frequenzänderung/Hz |
| NIT N9 | Nonylphenol-nona-ethylenglykolether | 80 |
| NIT N5,5 | Nonylphenol-penta/hexa-ethylenglykolether | 105 |
| NIT F1315 | C13/C15Fettalkohol-heptaethylenglykolether | 52 |
| AAT Semisuxol | Sulfobernsteinsäure-mono-C13/C15ester | 35 |

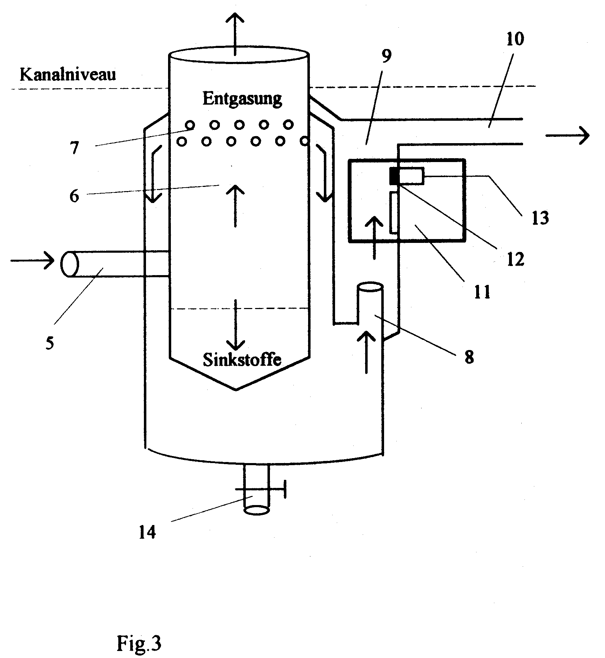

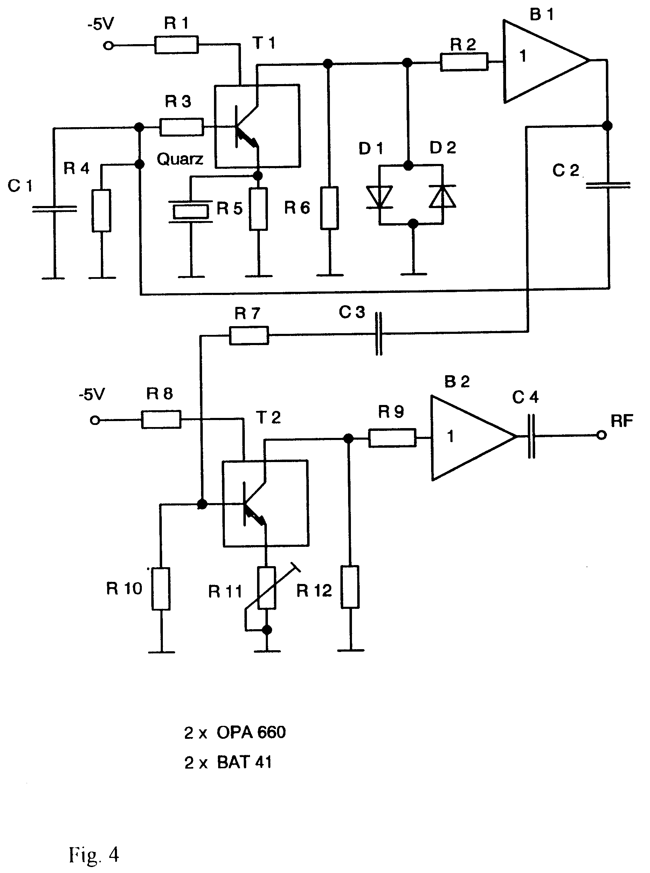

NIT können dabei in einem Bereich von 10 < X < 500 ppm, AAT in einem Konzentrationsbereich von 10 < X < 1200 ppm vermessen werden. Die Querempfindlichkeit zu sonstigen Abwasserinhaltsstoffen werden zu je 5 Hz an Glycolen für 60 ppm bzw. Dioctylphthalat für 4 ppm und CKW für 100 ppm gemessen.
Für eine in situ-Messung in einem Abwasserkanal zur Überwachung des Tensidgehaltes kommt eine Durchflußzelle nach Fig. 3 zum Einsatz. Durch den Eintragsstutzen 5 kommt das Wasser in die Kammer 6, in der eine Abtrennung der Schwebeteilchen und gleichzeitig die Entgasung der Flüssigkeit erfolgt. Durch kleine Löcher 7 fließt die Flüssigkeit über ein Rohr 8 in die Kammer 9 und dann durch ein weiteres Rohr 10 zum zeitgetakteten Auslauf. In der Kammer 9 befindet sich ein Multisensorsystem 11 mit einem gekapselten QMB-Sensor 12 und dem Oszillator 13 (Fig. 4) sowie einer Datenauswerteeinheit.
Für in situ-Messungen im Abwasserkanal müssen sowohl Gasblasen als auch Schwebteilchen von der Sensoroberfläche durch die speziell konzipierte Durchflußzelle vom QMB-Sensor 12 ferngehalten werden. Dies wird nicht nur durch das Abscheiden der Sink- und Schwebestoffe durch Beruhigungsschikanen erreicht, sondern außerdem dadurch, daß im Zeittakt von ca. 2 h die Probe durch die Anlage gesaugt wird, um die Filterwirkung in den Kammern 6 und 9 nicht zu überfordern. Die Reinigung der Anlage erfolgt über den Bodenablaß 14 oder über die Entgasungsöffnung.
In dem Multisensorsystem 11 befinden sich neben den gekoppelten QMB-Sensor noch weitere kommerzielle Sensoren für den pH-Wert, Temperatur und elektrische Leitfähigkeit, die ein multivalentes Sensorsystem darstellen. Durch multivariate Auswertung der Meßdaten mit Mitteln der Mustererkennung (Partitonierung und Neuronale Netze) reagiert der Multisensor spezifisch auf Änderungen eines Abwasserzustands durch die Anwesenheit von Tensiden und kann zur Charakterisierung des Abwassers eingesetzt werden.
Die Schaltung des Oszillators 13 ist identisch mit der Oszillatorschaltung in Fig. 4. Als Datenspeicher- und Auswerteeinheit wird eine speicherprogrammierbare Steuerung SPS eingesetzt.

**Patentansprüche**

1.  Verfahren zum Nachweis amphiphiler Stoffe in wäßriger Matrix im off line-oder on line-Betrieb ohne vorherige An- oder Abreicherungsoperationen dieser Stoffe,

    dadurch gekennzeichnet, daß

    die amphiphilen Stoffe auf der Oberfläche eines metallkontaktierten Schwingquarzes die Wasserschicht der wäßrigen Matrix verdrängen und die korzentrationsabhängige Masseänderung über eine Frequenzänderung des Schwingquarzes ein Sensorsignal erzeugt.

2.  Verfahren nach Anspruch 1, dadurch gekennzeichnet, daß die wäßrige Matrix als stationäre Strömung an der Oberfläche des Schwingquarzes vorbeigeführt wird.

3.  Vorrichtung zum Nachweis amphiphiler Stoffe in wäßriger Matrix nach den Ansprüchen 1 und 2 bestehend aus

    einer Durchflußzelle mit einem Flüssigkeitseintritt,
    einer Abscheidekammer, welche einen Auslaß für Sinkstoffe und einen Auslaß für Ausgasungen aufweist,
    einer Beruhigungskammer mit einem Flüssigkeitsaustritt,
    einem sich in der Beruhigungskammer befindlichen Multisensor mit einer Quarzkristall-Mikrobalance-Sensorzelle, deren metallkontaktierte Schwingquarzoberfläche sich mit dem amphiphilen Stoff in Kontakt befindet
    und einem an den Quarzkristall-Mikrobalance-Sensor angeschlossenen Oszillator sowie einer Auswerteeinheit.

4.  Vorrichtung nach Anspruch 3, dadurch gekennzeichnet, daß der Multisensor neben der Quarzkristall-Mikrobalance-Sensorzelle weitere Sensoren zur Messung des pH-Wertes, der elektrischen Leitfähigkeit, der Temperatur und/oder der Trübung enthält.

5.  Vorrichtung nach den Ansprüchen 3 und 4, dadurch gekennzeichnet, daß

    der Schwingtransistor (T1) mit seinem Emitter an eine Parallelschaltung aus einem Emitterwiderstand (R1) und dem Schwingquarz (Q) geschaltet ist, so daß die mit der Flüssigkeit in Berührung stehende Elektrode Massepotential besitzt,
    die Oszillatorschaltung außer dem Quarz keine frequenzbestimmenden Bauelemente besitzt,
    die antiparallel geschalteten Dioden (D1, D 2) galvanisch an den Kollektor des Schwingtransistors (T1) geschaltet sind,
    die Schaltung eine Leistungstreiberstufe bestehend aus dem Diamond-Transistor (T 2) und Buffer (B 2) enthält,
    die Treiberstufe kapazitiv mit den Ausgang des Buffers (B 1) verbunden ist.

3

4

1

Frequenzzähler
Datenspeicher

PC
Drucker

QMB-Sensoren

Oszillator

2

Fig.1

Fig. 2

Kanalniveau

Entgasung

Sinkstoffe

Fig.3

2 x OPA 660

2 x BAT 41

Fig. 4

Europäisches
Patentamt

**EUROPÄISCHER RECHERCHENBERICHT**

Nummer der Anmeldung

EP 96 10 8981

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y,D | SENSORS AND ACTUATORS B, Bd. 18-19, 1994, LAUSANNE CH, Seiten 518-522, XP000195583 J. AUGE ET AL.: * Zusammenfassung; Abbildung 6 * * Seite 521 * | 1,2 | G01N27/00 G01N33/18 |
| A | --- | 5 | |
| Y | WO-A-85 04254 (FÖRSVARETS FORSKNINGSANSTALT) * Seite 2, Zeile 29 - Seite 4, Zeile 16 * --- | 1,2 | |
| A | DE-A-42 27 727 (BUNA AG) * Seite 3, Zeile 60 - Zeile 62 * --- | 4 | |
| A,D | EXPERIMENTAL TECHNIQUE OF PHYSICS, Bd. 40, Nr. 1, 1994, Seiten 27-34, XP000195586 J. AUGE ET AL.: * Abbildung 3 * --- | 5 | |
| A,D | ANALYTICAL CHEMISTRY, Bd. 63, Nr. 3, 1.Februar 1991, COLUMBUS US, Seiten 203-207, XP000577311 Y. OKAHATA ET AL.: * Seite 203 * --- | 1 | **RECHERCHIERTE SACHGEBIETE (Int.Cl.6)** G01N |
| A | US-A-3 856 466 (H. M. CRAWFORD) * Zusammenfassung * --- | 1 | |
| A | US-A-5 201 215 (V. E. GRANSTAFF ET AL.) * Zusammenfassung * --- | 1 | |
| A,D | EP-A-0 473 901 (FRAUNHOFER-GESELLSCHAFT ZUR FÖRDERUNG DER ANGEWANDTEN FORSCHUNG E.V.) * Abbildungen 1-3 * ----- | 5 | |

Der vorliegende Recherchenbericht wurde für alle Patentansprüche erstellt

| Recherchenort | Abschlußdatum der Recherche | Prüfer |
|---|---|---|
| BERLIN | 7.Oktober 1996 | Brison, O |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus andern Gründen angeführtes Dokument
..........................................................................................
& : Mitglied der gleichen Patentfamilie, übereinstimmendes
     Dokument

EPO FORM 1503 03.82 (P04C03)